Europäisches Patentamt

European Patent Office

Office européen des brevets

(11) Publication number: **0 473 045 A1**

## EUROPEAN PATENT APPLICATION

(21) Application number: **91113930.1**

(22) Date of filing: **20.08.91**

(51) Int. Cl.5: **A61M 25/00, F16L 11/08**

(30) Priority: **24.08.90 JP 88782/90**

(43) Date of publication of application:
**04.03.92 Bulletin 92/10**

(84) Designated Contracting States:
**BE DE FR GB IT SE**

(71) Applicant: **JUNKOSHA CO. LTD.**
**25-25, Miyasaka 2-chome**
**Setagaya-ku Tokyo 156(JP)**

(72) Inventor: **Ikeda, Shingo**
**Nakayama 393**
**Hannoh, Saitama(JP)**

(74) Representative: **Kehl, Günther, Dipl.-Phys. et al**
**Patentanwälte Hagemann & Kehl Ismaninger Strasse 108 Postfach 86 03 29**
**W-8000 München 86(DE)**

(54) **Medical tube.**

(57) Disclosed herein is a medical tube (1) which comprises a surface-treated inner tube (2) of fluoroplastic, a metal wire (3) spirally wound round the inner tube (2), and an outer covering (4) of polyurethane placed outside the spirally wound metal wire (3), said outer covering (4) being bonded to and integral with the inner tube (2) through the interstices of the spirally wound metal wire (3). It does not collapse easily when bent, because the spirally wound metal wire (3) reinforces the tube wall. It can be bent with a smaller bending radius than the conventional one. It can be made in smaller diameter because it is possible to reduce the wall thickness without any fear of its collapsing. These advantages contribute to reducing the diameter of the inserting part of an endoscope.

## Background of the Invention

### 1. Field of the Invention:

The present invention relates to a medical tube suitable for use as an endoscope and catheter.

### 2. Description of the Prior Art:

An endoscope for medical use is made up of a manipulating part and a long flexible inserting part to be inserted into the patient's body. The manipulating part is provided with an eyepiece, a knob to bend the inserting part, and an inlet for the insertion of forceps and other apparatus to treat the affected part. The inserting part is a flexible tube, with one end thereof attached to the manipulating part, which contains a light-conveying member (such as image guide and light guide), a tube that provides a passageway for forceps, an air tube, a water tube, and a wire to bend the inserting part. In other words, the inserting part of an endoscope contains several channel tubes. These tubes are conventionally made of microporous polytetrafluoroethylene so that they have the desired flexibility and inside slipperiness, as disclosed in Japanese Patent Laid-open Nos. 109535/1990 and 126827/1990.

A disadvantage of the above-mentioned conventional medical tube is the low resistance to collapse which results from its open-cell microporous structure. This is true particularly in the case where it has a thin wall thickness. Therefore, there is a limit in reducing the wall thickness and bending radius. Moreover, the open-cell microporous structure poses a problem associated with the growth of infectious microbes.

## Summary of the Invention

The present invention was completed to eliminate the above-mentioned disadvantages involved in the prior art technology. It is an object of the present invention to provide a medical tube which has a smooth inside surface and can be bent with a small bending radius. The medical tube is suitable for use as a medical instrument such as an endoscope which is inserted into the patient's body.

The present invention is embodied in a medical tube which comprises a surface-treated inner tube of fluoroplastic, a metal wire spirally wound round the inner tube, and an outer covering of polyurethane placed outside the spirally wound metal wire, said outer covering being bonded to and integral with the inner tube through the interstices of the spirally wound metal wire.

According to the present invention, the medical tube is produced by spirally winding a metal wire at a prescribed pitch round an inner tuber of fluoroplastic and then covering the spiral of metal wire with polyurethane by melt extrusion. During extrusion, the polyurethane fills the interstices of the spirally wound metal wire so that the outer covering comes into close contact with the inner tube. Since the inner tube is surface-treated to reduce its abhesiveness, the extruded molten polyurethane becomes integral with the inner tube through fusion bonding. The spirally wound metal wire prevents the tube from collapsing when the tube is bent with a small bending radius. Moreover, the inner tube, which is made of non-porous fluoroplastic, prevents the growth of infectious microbes and provides a good slipperiness which permits the smooth insertion of forceps etc.

## Brief Description of the Drawings

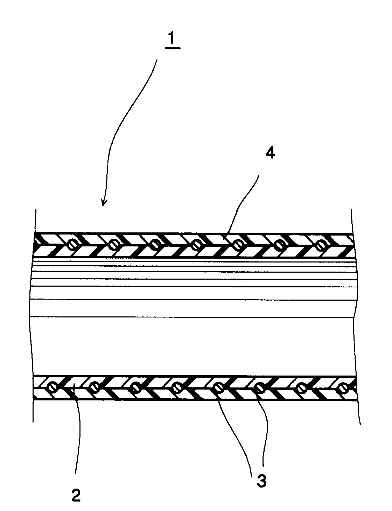

Fig. 1 is a longitudinal sectional view showing an embodiment of the medical tube according to the present invention.

## Detailed Description of the Preferred Embodiments

The invention will be described in more detail with reference to Fig. 1 which is a longitudinal sectional view showing the medical tube according to the present invention. The medical tube 1 is made up of an inner tube 2 of tetrafluoroethylene-hexafluoropropylene copolymer, a metal wire 3 (such as stainless steel wire having an adequate stiffness) spirally wound (with a coarse pitch) round the inner tube 2, and an outer covering 4 of polyurethane which is integral with the inner tube 2 through the interstices of the spirally wound metal wire 3.

Although the inner tube 2 originally has an abhesive surface, it can be made adhesive by surface treatment with metallic sodium dispersed in naphthalene or by surface roughening with plasma irradiation. (The surface treatment brings about chemical reactions which release and polarize fluorine atoms on the surface.) The inner tube 2 is spirally wound with a metal wire, and then covered with molten polyurethane by extrusion to form the outer covering 4. The molten polyurethane passes through the interstices of the coil of the metal wire, reaching the surface of the inner tube 2. Owing to the treated surface of the inner tube, the polyurethane outer covering becomes integral with the inner tube through fusion bonding.

In other words, according to the present invention, it is possible to integrate the three components with one another without resorting to an adhesive. Therefore, the medical tube of the present invention is not subject to leaching, unlike the conventional one which contains an adhesive. Moreover, polyurethane is suitable for the outer covering 4 because it has good mechanical strength, flexibility, and non-toxic properties, and it easily bonds when melted.

Being constructed as mentioned above, the medical tube of the present invention offers the following advantages. It does not collapse easily when bent, because the spirally wound metal wire reinforces the tube wall. It can be bent with a smaller bending radius than the conventional one. It can be made in smaller diameter because it is possible to reduce the wall thickness without any fear of its collapsing. (These advantages contribute to reducing the diameter of the inserting part of an endoscope.) It has the inner tube 2 of non-porous fluoroplastic which prevents the growth of infectious microbes and provides a smooth inner surface having a small coefficient of friction. The smooth inner surface is convenient for the insertion and movement of forceps in the tube.

The foregoing is concerned with an embodiment in which the inner tube 2 is made of tetrafluoroethylene-hexafluoropropylene copolymer; but the present invention is not limited to this embodiment. It is possible to use other fluoroplastics such as tetrafluoroethylene resin, tetrafluoroethylene-perfluoroalkyl vinyl ether copolymer resin, and ethylene-tetrafluoroethylene copolymer resin. It is also possible to replace the stainless steel wire by piano wire.

The medical tube of the present invention is suitable for use as the channel tubes of an endoscope through which forceps etc. are passed. It can also be used as the flexible tube (inserting part) of an endoscope that contains the channel tubes and other members. It will find use as a catheter. The possible application depends on the wall thickness and the diameter, winding pitch, and material of the metal wire coil.

## Claims

1. A medical tube which comprises a surface-treated inner tube (2) of fluoroplastic, a metal wire (3) spirally wound round the inner tube (2), and an outer covering (4) of polyurethane placed outside the spirally wound metal wire (3), said outer covering (4) being bonded to and integral with the inner tube (2) through the interstices of the spirally wound metal wire (3).

2. Medical tube according to claim 1, wherein the inner tube (2) is made of non-porous material.

3. Medical tube according to anyone of the preceding claims, wherein the inner tube (2) is made of a fluoroalkylene-copolymer.

4. Medical tube according to anyone of the preceding claims, wherein said outer covering (4) is bonded to said inner tube (2) free of adhesive.

5. Method for manufacturing of the medical tube according to anyone of the preceding claims, wherein a metal wire (3) is being wound round the inner tube (2), the inner tube (2) is being surface treated and the inner tube together with the metal wire (3) is being covered by the outer covering (4) by melt extrusion.

6. Method according to claim 5, wherein the surface treatment is made by metallic sodium dispersed in naphthalene.

7. Method according to claim 5, wherein the surface treatment is made by plasma irradiation.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-3 568 660 (CHAMBERS)<br>* column 2, line 10 - line 19 *<br>* column 4, line 15 - line 20 *<br>* column 4, line 29 - line 31 *<br>* column 4, line 34 - line 38; figures 4,6,7 * | 1,2 | A61M25/00<br>F16L11/08 |
| A | | 5 | |
| Y | DE-A-3 525 731 (LEMM)<br>* column 4, line 29 - line 40; claims 9-11 * | 1,2 | |
| A | GB-A-2 008 223 (AUTOMATION INDUSTRIES INC.)<br>* page 1, line 18 - line 20 *<br>* page 2, line 49 - line 61 *<br>* page 2, line 76 - line 103 *<br>* page 3, line 70 - line 83; figure 10 * | 1,3-5 | |
| A | US-A-3 814 138 (COURTOT)<br>* column 3, line 8 - line 10 *<br>* column 3, line 21 - line 23; figure 1 * | 1,5 | |
| A | WORLD PATENTS INDEX LATEST<br>Section PQ, Week 8911,<br>Derwent Publications Ltd., London, GB;<br>Class Q67, AN 89-083201<br>& SU-A-1 420 297 (YUZHVODOPROVOD)<br>* abstract * | 6 | TECHNICAL FIELDS SEARCHED (Int. Cl.5)<br><br>A61M<br>F16L |
| A | GB-A-2 102 788 (HERAEUS QUARZSCHMELZE GMBH)<br>* page 1, line 95 - line 96 * | 7 | |

The present search report has been drawn up for all claims

| Place of search | Date of completion of the search | Examiner |
|---|---|---|
| THE HAGUE | 29 NOVEMBER 1991 | SEDY R. |